# EUROPEAN PATENT APPLICATION

(11) **EP 3 037 539 A1**
(43) Date of publication of application: **29.06.2016**
(21) Application number: 14838021.5
(22) Date of filing: 23.07.2014
(51) Int. Cl.: C12N 15/47, A61K 39/205

(54) **RECOMBINANT VACCINE AGAINST VIRAL HEMORRHAGIC SEPTICEMIA VIRUS**

(30) Priority: 23.08.2013 ES 201331272
(71) Applicant: Universidad Miguel Hernandez De Elche, 03202 Elche (Alicante) (ES)
(72) Inventor: ESTEPA PEREZ, Maria Amparo, E-03202 Elche (Alicante) (ES); MARTINEZ LOPEZ, Alicia, E-03202 Elche (Alicante) (ES)
(74) Representative: Pons Ariño, Angel
(86) International application number: PCT/ES2014/070592
(87) International publication number: WO 2015/025068

(57) **Abstract**

The present invention relates to a recombinant deoxyribonucleic acid vaccine comprising the coding sequence for the ectodomain of the glycoprotein G of the viral haemorrhagic septicaemia virus (VHSV), the coding sequence for a signal peptide of pleurocidin of *Limanda limanda* and the promoter of β-actin of carp for the treatment and / or prevention of viral haemorrhagic septicaemia caused by VHSV. The vaccine of the invention provides protection when administered both intramuscularly and intraperitoneally.

SEQUENCE LISTING

<110>  Universidad Miguel Hernández de Elche

<120>  Recombinant vaccine against the viral haemorrhagic septicaemia virus

<130>  ES2047.14

<160>  8

<170>  PatentIn version 3.5

<210>  1
<211>  1327
<212>  DNA
<213>  Artificial sequence

<220>
<223>  cDNA of the ectodomain of glycoprotein G of VHSV

<400>  1
cagatcactc aacgacctcc ggtcgaaaac atctcgacgt accatgcaga ctgggacact      60

ccgctataca ctcatccctc caactgcagg gacgattcct ttgtcccgat tcgaccagct     120

caactcaggt gtcctcatga atttgaagac ataaacaagg gactggtttc cgtcccaacc     180

aggatcatcc atctcccgct atcggtcacc agcgtctccg cagtagcgag tggccactac     240

ctgcacagag tgacttatcg agtcacctgt tcgaccagct tctttggagg gcaaaccatc     300

gaaaagacca tcttggaggc gaaactgtct cgtcaggagg ccacagacga ggcaagcaag     360

gatcacgagt acccgttctt ccctgaaccc tcctgcatct ggatgaaaaa caatgtccat     420

aaggacataa ctcactatta caagacccca aaaacagtat cggtggatct ctacagcagg     480

aaatttctca accctgattt catagagggg gtttgcacaa cctcgccctg tcaaactcat     540

tggcagggag tctattgggt cggtgccaca cctaaagccc attgccccac gtcggaaaca     600

ctagaaggac acctgttcac caggacccat gatcacaggg tggtcaaggc aattgtggca     660

ggccatcatc cctgcgggac tcacaatggc atgcacagtg acattctgcg ggacagaatg     720

gatcaagact gacctggggg acctgatcca ggtgacagga ccggggggca cgaggaaact     780

gactccaaac aagtgtgtca ataccgatat ccagatgagg ggggcaacag acgacttttc     840

ttatctcaac catctcatca ccaacatggc tcaaagaacc gagtgcctag atgcccatag     900

tgatatcacc gcttctggga aagtatcctc atttctcctc tcaaagtttc gtcccagcca     960

ccctggacct ggcaaggcac actatcttct cgacggtcaa atcatgcgag gtgactgtga    1020

ctatgaggca gtagtcagca tcaactacaa tcgcgctcaa tacaagacgg tgaacaacac    1080

atggaaatca tggaaacggg tagacaacaa cacagacggg tacgatggga tgatatttgg    1140

ggacaaattg atcatcccgg acatcgaaaa gtatcagagt gtctatgaca gtggaatgct    1200

cgttcaaaga aaccttgtgg aagtccctca tctgagcatt gtgtttgtct ccaacacatc    1260

tgatctttcc actaatcaca tccacaccaa cctaatccct tcggattggt cattcaactg 1320

gagtctt 1327

<210> 2
<211> 71
<212> DNA
<213> Limanda limanda

<400> 2
atgaagttca ctgccacctt cctcgtgatg ttcatcttcg tcctcatggt tgaacctgga 60

gagtgtggtg c 71

<210> 3
<211> 2533
<212> DNA
<213> Artificial sequence

<220>
<223> beta-actin promoter

<400> 3
ttcacacagg aaacagctat gaccatgatt acgccaagct tttagacctt cttacttttg 60

gggattatat aagtattttc tcaataaata tctcatatct tactgtggtt taactgctga 120

atctaaaatt ttaatacaaa agtagttata tttgttgtac attgtaaact ataacttaac 180

ttcagtttca gagaaactca tgtgctcaaa atgtaaaaaa agtttcctgt taaatatttt 240

gtaaatgtat tgaagacaaa ataagaaaaa aaaaaatata agccactaaa tcacactgtc 300

cttggtatca gcaagagatt ctgacataat cagctgtttt tgtttattac tgccattgaa 360

ggccatgtgc attagtccca agttacacat taaaaagtca catgtagctt accaacatca 420

gtgctgttca agcacagcct catctactat tcaaactgtg gcaccatcta aaatatgcca 480

gaattttttt atttaatgaa tttgaccctg aaatatgtat taatatcact cctgtgattt 540

ttttgtaatc agcttacaat tacaggaatg caagcctgat tcattacaag tttcactaca 600

ctttctctga caacatcacc tactgaactc agaccagcta gttgctcctt aagtatacaa 660

tcatgtcact aatcctcatt tcaatgaaaa atacccctat tgtacttggt acttggtaga 720

taaccacaga gcagtattat gccattattg tgaatacaat aagaggtaaa tgacctacag 780

agctgctgct gctgttgtgt tagattgtaa acacagcaca ggatcaagga ggtgtccatc 840

actatgacca atactagcac tttgcacagg ctctttgaaa ggctgaaaag agccttattg 900

gcgttatcac aacaaaatac gcaaatacgg aaaacaacgt attgaacttc gcaaacaaaa 960

aacagcgatt ttgatgaaaa tcgcttaggc cttgctcttc aaacaatcca gcttctcctt 1020

ctttcactct caagttgcaa gaagcaagtg tagcaatgtg cacgcgacag ccgggtgtgt 1080

gacgctggac caatcagagc gcagagctcc gaaagtttac cttttatggc tagagccggc 1140

atctgccgtc atataaaaga gcgcgcccag cgtctcagcc tcactttgag ctcctccaca 1200

cgcagctagt gcggaatatc atctgcctgt aacccattct ctaaagtcga caaacccccc 1260

caaacctaag gtgagttgat ctttaagctt tttacatttt cagctcgcat atatcaattc 1320

gaacgtttaa ttagaatgtt taaataaagc tagattaaat gattaggctc agttaccggt 1380

cttttttttc tcatttacgt gcgaactctg cttaaactct agttattctt tattaatatg 1440

tggttatttt tatatatgta tgttatcata actgtactgg ctatgtcagg tggtaatgac 1500

tgtaacgtta cgttactcgt tgtaggcacg acattgaatg ggccggtgtt gaaataagtc 1560

ttcaacccct tttaacctca aaatgtgctc tggttaacaa ggattttaac agctatcagt 1620

atgactgtgc ggttttaaag ccgttagtga ggcacgttgc acacttgatg gatggccgga 1680

atgggaagtt ctttatgcag gcagtgctgc agcagggtgt gacctacttt agctaacgtt 1740

agccggctaa ccagcattca tctgccggta acttgagtct aatattctct atgtgatatc 1800

gaagtgatca aagacacgtc tgttagctca ctttaaccaa ctgtagtgaa aaatagcgca 1860

gtgtgcagcc cttcaagtct ttcatttagg ctgattattc aatcatttta ttaactatta 1920

acgcgttact aaacgtaagg taacgtagtc agtttttaat aactggtgaa aagtactggt 1980

tgggtttaaa tggtgactta taattgtgtt ggagggggaa acctttttga taaaggctat 2040

ataatctcaa atgaatgggc tgaggatggt gttcacaggt gctttagtga agtccgctcg 2100

tgaagagtcg ctgaagtgac tgcagatctg tagcgcatgc gttttggcag acggccgttg 2160

aaattcggtt gagtaattga taccaggtga ggctagagga tgtagaaatt catttgtgta 2220

gaatttaggg agtggcctgg cgtgatgaat gtcgaaatcc gttccttttt actgaaccct 2280

atgtctctgc tgagtgccac accgccggca caaagcgtct caaaccattg ccttttatgg 2340

taataatgag aatgcagagg gacttccttt gtctggcaca tctgaggcgc gcattgtcac 2400

actagcaccc actagcggtc agactgcaga caaacaggaa gctgactcca catggtcaca 2460

tgctcactga agtgttgact tccctgacag ctgtgcactt tctaaaccgg ttttctcatt 2520

catttacagt tca 2533

<210> 4
<211> 3941
<212> DNA
<213> Artificial sequence

<220>
<223> Recombinant nucleic acid

<400> 4
ttcacacagg aaacagctat gaccatgatt acgccaagct tttagacctt cttacttttg 60

gggattatat aagtattttc tcaataaata tctcatatct tactgtggtt taactgctga 120

atctaaaatt ttaatacaaa agtagttata tttgttgtac attgtaaact ataacttaac 180

ttcagtttca gagaaactca tgtgctcaaa atgtaaaaaa agtttcctgt taaatatttt 240

gtaaatgtat tgaagacaaa ataagaaaaa aaaaaatata agccactaaa tcacactgtc 300

cttggtatca gcaagagatt ctgacataat cagctgtttt tgtttattac tgccattgaa 360

ggccatgtgc attagtccca agttacacat taaaaagtca catgtagctt accaacatca 420

gtgctgttca agcacagcct catctactat tcaaactgtg gcaccatcta aaatatgcca 480

gaattttttt atttaatgaa tttgaccctg aaatatgtat taatatcact cctgtgattt 540

ttttgtaatc agcttacaat tacaggaatg caagcctgat tcattacaag tttcactaca 600

ctttctctga caacatcacc tactgaactc agaccagcta gttgctcctt aagtatacaa 660

tcatgtcact aatcctcatt tcaatgaaaa atacccctat tgtacttggt acttggtaga 720

taaccacaga gcagtattat gccattattg tgaatacaat aagaggtaaa tgacctacag 780

agctgctgct gctgttgtgt tagattgtaa acacagcaca ggatcaagga ggtgtccatc 840

actatgacca atactagcac tttgcacagg ctctttgaaa ggctgaaaag agccttattg 900

gcgttatcac aacaaaatac gcaaatacgg aaaacaacgt attgaacttc gcaaacaaaa 960

aacagcgatt ttgatgaaaa tcgcttaggc cttgctcttc aaacaatcca gcttctcctt 1020

ctttcactct caagttgcaa gaagcaagtg tagcaatgtg cacgcgacag ccgggtgtgt 1080

gacgctggac caatcagagc gcagagctcc gaaagtttac cttttatggc tagagccggc 1140

atctgccgtc atataaaaga gcgcgcccag cgtctcagcc tcactttgag ctcctccaca 1200

cgcagctagt gcggaatatc atctgcctgt aacccattct ctaaagtcga caaacccccc 1260

caaacctaag gtgagttgat ctttaagctt tttacatttt cagctcgcat atatcaattc 1320

gaacgtttaa ttagaatgtt taaataaagc tagattaaat gattaggctc agttaccggt 1380

cttttttttc tcatttacgt gcgaactctg cttaaactct agttattctt tattaatatg 1440

tggttatttt tatatatgta tgttatcata actgtactgg ctatgtcagg tggtaatgac 1500

tgtaacgtta cgttactcgt tgtaggcacg acattgaatg ggccggtgtt gaaataagtc 1560

ttcaacccct tttaacctca aaatgtgctc tggttaacaa ggattttaac agctatcagt 1620

atgactgtgc ggttttaaag ccgttagtga ggcacgttgc acacttgatg gatggccgga 1680

atgggaagtt ctttatgcag gcagtgctgc agcagggtgt gacctacttt agctaacgtt 1740

agccggctaa ccagcattca tctgccggta acttgagtct aatattctct atgtgatatc 1800

gaagtgatca aagacacgtc tgttagctca ctttaaccaa ctgtagtgaa aaatagcgca 1860

gtgtgcagcc cttcaagtct ttcatttagg ctgattattc aatcatttta ttaactatta 1920

acgcgttact aaacgtaagg taacgtagtc agtttttaat aactggtgaa aagtactggt 1980

tgggtttaaa tggtgactta taattgtgtt ggagggggaa acctttttga taaaggctat 2040

ataatctcaa atgaatgggc tgaggatggt gttcacaggt gctttagtga agtccgctcg 2100

tgaagagtcg ctgaagtgac tgcagatctg tagcgcatgc gttttggcag acggccgttg 2160

aaattcggtt gagtaattga taccaggtga ggctagagga tgtagaaatt catttgtgta 2220

gaatttaggg agtggcctgg cgtgatgaat gtcgaaatcc gttccttttt actgaaccct 2280

atgtctctgc tgagtgccac accgccggca caaagcgtct caaaccattg ccttttatgg 2340

taataatgag aatgcagagg gacttccttt gtctggcaca tctgaggcgc gcattgtcac 2400

actagcaccc actagcggtc agactgcaga caaacaggaa gctgactcca catggtcaca 2460

tgctcactga agtgttgact tccctgacag ctgtgcactt tctaaaccgg ttttctcatt 2520

catttacagt tcagccggta ccgagctcgg atccactagt aacggccgcc agtgtgctgg 2580

aattcgccct tatgaagttc actgccacct tcctcgtgat gttcatcttc gtcctcatgg 2640

ttgaacctgg agagtgtggt gcggccgctc agatcactca acgacctccg gtcgaaaaca 2700

tctcgacgta ccatgcagac tgggacactc cgctatacac tcatccctcc aactgcaggg 2760

acgattcctt tgtcccgatt cgaccagctc aactcaggtg tcctcatgaa tttgaagaca 2820

taaacaaggg actggtttcc gtcccaacca ggatcatcca tctcccgcta tcggtcacca 2880

gcgtctccgc agtagcgagt ggccactacc tgcacagagt gacttatcga gtcacctgtt 2940

cgaccagctt ctttggaggg caaaccatcg aaaagaccat cttggaggcg aaactgtctc 3000

gtcaggaggc cacagacgag gcaagcaagg atcacgagta cccgttcttc cctgaaccct 3060

cctgcatctg gatgaaaaac aatgtccata aggacataac tcactattac aagaccccaa 3120

aaacagtatc ggtggatctc tacagcagga aatttctcaa ccctgatttc atagaggggg 3180

tttgcacaac ctcgccctgt caaactcatt ggcagggagt ctattgggtc ggtgccacac 3240

ctaaagccca ttgccccacg tcggaaacac tagaaggaca cctgttcacc aggacccatg 3300

atcacagggt ggtcaaggca attgtggcag gccatcatcc ctggggactc acaatggcat 3360

gcacagtgac attctgcggg acagaatgga tcaagactga cctgggggac ctgatccagg 3420

tgacaggacc ggggggcacg aggaaactga ctccaaacaa gtgtgtcaat accgatatcc 3480

agatgagggg ggcaacagac gacttttctt atctcaacca tctcatcacc aacatggctc 3540

aaagaaccga gtgcctagat gcccatagtg atatcaccgc ttctgggaaa gtatcctcat 3600

ttctcctctc aaagtttcgt cccagccacc ctggacctgg caaggcacac tatcttctcg 3660

acggtcaaat catgcgaggt gactgtgact atgaggcagt agtcagcatc aactacaatc 3720

gcgctcaata caagacgatg aacaacacat ggaaatcatg gaaacgggta gacaacaaca 3780

cagacgggta cgatgggatg atatttgggg acaaattgat catcccggac atcgaaaagt 3840

atcagagtgt ctatgacagt ggaatgctcg ttcaaagaaa ccttgtggaa gtccctcatc 3900

tgagcattgt gtttgtctcc aacacatctg atctttcctg a 3941

<210>  5
<211>  442
<212>  PRT
<213>  Artificial sequence

<220>
<223>  Ectodomain of glycoprotein G of VHSV

<400>  5

Val Ile Leu Ile Ile Ile Ile Lys Ser Thr Thr Pro Gln Ile Thr Gln
1               5                   10                  15

Arg Pro Pro Val Glu Asn Ile Ser Thr Tyr His Ala Asp Trp Asp Thr
            20                  25                  30

Pro Leu Tyr Thr His Pro Ser Asn Cys Arg Asp Asp Ser Phe Val Pro
        35                  40                  45

Asn Arg Pro Ala Gln Leu Arg Cys Pro His Glu Phe Glu Asp Ile Asn
    50                  55                  60

Lys Gly Leu Val Ser Val Pro Thr Arg Ile Ile His Leu Pro Leu Ser
65                  70                  75                  80

Val Thr Ser Val Ser Ala Val Ala Ser Gly His Tyr Leu His Arg Val
                85                  90                  95

Thr Tyr Arg Val Thr Cys Ser Thr Ser Phe Phe Gly Gly Gln Thr Ile
            100                 105                 110

Glu Lys Thr Ile Leu Glu Ala Lys Leu Ser Arg Gln Glu Ala Thr Asp
        115                 120                 125

Glu Ala Ser Lys Asp His Glu Tyr Pro Phe Phe Pro Glu Pro Ser Cys
    130                 135                 140

Ile Trp Met Lys Asn Asn Val His Lys Asp Ile Thr His Tyr Tyr Lys
145                 150                 155                 160

Thr Pro Lys Thr Val Ser Val Asp Leu Tyr Ser Arg Lys Phe Leu Asn
                165                 170                 175

Pro Asp Phe Ile Glu Gly Val Cys Thr Thr Ser Pro Cys Gln Thr His
            180                 185                 190

Trp Gln Gly Val Tyr Trp Val Gly Ala Thr Pro Lys Ala His Cys Pro
        195                 200                 205

Thr Ser Glu Thr Leu Glu Gly His Leu Phe Thr Arg Thr His Asp His
    210                 215                 220

Arg Val Val Lys Ala Ile Val Ala Gly His His Pro Trp Gly Leu Thr
225                 230                 235                 240

Met Ala Cys Thr Val Thr Phe Cys Gly Thr Glu Trp Ile Lys Thr Asp
                245                 250                 255

Leu Gly Asp Leu Ile Gln Val Thr Gly Pro Gly Gly Thr Arg Lys Leu
            260                 265                 270

Thr Pro Asn Lys Cys Val Asn Thr Asp Ile Gln Met Arg Gly Ala Thr
        275                 280                 285

Asp Asp Phe Ser Tyr Leu Asn His Leu Ile Thr Asn Met Ala Gln Arg
    290                 295                 300

Thr Glu Cys Leu Asp Ala His Ser Asp Ile Thr Ala Ser Gly Lys Val
305                 310                 315                 320

Ser Ser Phe Leu Leu Ser Lys Phe Arg Pro Ser His Pro Gly Pro Gly
                325                 330                 335

Lys Ala His Tyr Leu Leu Asp Gly Gln Ile Met Arg Gly Asp Cys Asp
            340                 345                 350

Tyr Glu Ala Val Val Ser Ile Asn Tyr Asn Arg Ala Gln Tyr Lys Thr
        355                 360                 365

Met Asn Asn Thr Trp Lys Ser Trp Lys Arg Val Asp Asn Asn Thr Asp
    370                 375                 380

Gly Tyr Asp Gly Met Ile Phe Gly Asp Lys Leu Ile Ile Pro Asp Ile
385                 390                 395                 400

Glu Lys Tyr Gln Ser Val Tyr Asp Ser Gly Met Leu Val Gln Arg Asn
                405                 410                 415

Leu Val Glu Val Pro His Leu Ser Ile Val Phe Val Ser Asn Thr Ser
            420                 425                 430

Asp Leu Ser Thr Asn His Ile His Thr Asn
        435                 440

<210>  6
<211>  22

<212>  PRT
<213>  Limanda limanda

<400>  6

Met Lys Phe Thr Ala Thr Phe Leu Val Met Phe Ile Phe Val Leu Met
1               5                   10                  15

Val Glu Pro Gly Glu Cys
            20

<210>  7
<211>  27
<212>  DNA
<213>  Artificial sequence

<220>
<223>  Direct primer for the detection of the glycoprotein G of VHSV

<400>  7
cgaccagctc aactcaggtg tcctcat                                          27

<210>  8
<211>  27
<212>  DNA
<213>  Artificial sequence

<220>
<223>  Reverse primer for the detection of the glycoprotein G of VHSV

<400>  8
ggtgactcga taagtcactc tgtgcac                                          27

## Description

The present invention relates to a recombinant deoxyribonucleic acid vaccine comprising the coding sequence for the ectodomain of glycoprotein G of the viral haemorrhagic septicaemia virus (VHSV), the coding sequence for a signal peptide of pleurocidin of *Limanda limanda* and the promoter of β-actin of carp (*Cyprinus carpio*), for use against VHSV. The vaccine of the invention is useful in both intramuscular and intraperitoneal administration. Therefore, the invention may be encompassed in the field of Biomedicine.

### STATE OF THE ART

Among the diseases affecting fish, haemorrhagic septicaemia caused by VHSV, a virus of the *Rhabdoviridae* family, affecting for example salmonids, is causing considerable losses in the aquaculture sector (Gómez-Casado, E et al . 2011 Vaccine 29:2657-2671). Strategies developed to produce vaccines against rhabdovirus include the generation of recombinant DNA vaccines.

Recombinant deoxyribonucleic acid (DNA) vaccines are one of the most important tools developed in the field of vaccinology and providing the genes encoding an antigen instead of directly administering the antigen. Therefore, it is expected that, because of their simplicity and low production cost, they will soon be transferred to the line of development of pharmaceuticals. In fact, four DNA vaccines have recently been approved in the area of veterinary medicine. However, DNA vaccines are still facing various problems related to safety, antigenic immunogenicity, administration and stability, which slow down their worldwide licensing.

With regard to DNA vaccines, it is known that the routes of administration can play a key role in the magnitude and quality of the triggering of the immune response. This is the case of the DNA vaccine against the infectious hematopoietic necrosis rhabdovirus (IHNV) (Apex-IHN, *Aqua Health Ltd,* Canada). This vaccine, administered intramuscularly (i.m.) induces protective immunity in farmed salmon (85-98% protection), however, protection is only partial when administered intraperitoneally (i.p.) (Corbeil S. et al. 2000 Fish Shelfish Immunol 10:711-723; McLauchan PE et al 2003 Fish Shelfish Immunol 15:39-50).

The DNA vaccine against IHNV consists of a plasmid vector encoding the sequence of complementary DNA (cDNA) of the virus surface antigen, the glycoprotein G (gpG) anchored to the membrane, under the transcriptional control of the cytomegalovirus (CMV) promoter. It has been proposed that the transfection of antigen-presenting cells (APC), as with somatic cells, does not occur in the intraperitoneal cavity as efficiently as in muscle cells. In this case, reduced availability of antigen would not be sufficient to produce an optimal immune response, which may be the cause of the low efficiency of DNA vaccines administered intraperitoneally (i.p.). Intraperitoneal administration is an administration route commonly used for vaccination of fish.
For this reason, there is a need to develop a vaccine that works intraperitoneally, so that it can be included in the formulations of multivalent vaccines used in salmonid fish farms, allowing administration in a single injection.

### DESCRIPTION OF THE INVENTION

The technical problem solved by the invention is to provide a recombinant DNA vaccine conferring protection by intraperitoneal administration and also intramuscular administration for the treatment and / or prevention of viral haemorrhagic septicaemia caused by VHSV in fish.

The vaccine of the invention induces protective immunity regardless of the route of administration. The vaccine of the invention comprises recombinant deoxyribonucleic acid comprising the coding sequence for the ectodomain of glycoprotein G of the viral haemorrhagic septicaemia virus (preferably the strain VHSV_{07.71}), the coding sequence for a signal peptide of *Limanda limanda* pleurocidin and the promoter of β-actin of carp. For the development thereof, two alternative constructs to gpG1-507 of VHSV (wild gpG, membrane anchored) were conducted, on the one hand, a soluble gpG (gpG1-462) and on the other hand, a secretable form (gpGLmPle20-462). After the *in vivo* experiments, it was found that only the secretable form of gpG conferred protective immunity against VHSV by both routes of administration, intramuscular and intraperitoneal, this being the first time a DNA vaccine administered intraperitoneally is successful. In addition, this new DNA vaccine also provided protection when administered in conjunction with an oil adjuvant, therefore, this vaccine can be included in the formulation of injectable multivalent vaccines in fish. Moreover, a large recruitment of B cells was observed expressing immunoglobulins, especially IgT plus T cells expressing T-bet at short post-immunization times, mostly in the fish immunized with the secretable form of gpG. This may indicate that the subcellular localization of antigen expressed in cells *in vivo* may be an important factor in terms of how DNA vaccines prioritize the immune response.

As described herein, a first aspect of the invention relates to recombinant nucleic acid comprising a subunit (a) comprising the sequence SEQ ID NO:1 and a subunit (b) which comprises the sequence SEQ ID NO:2. Preferably the subunit (b) is linked to the 5' end of the subunit (a). In a preferred embodiment, the nucleic acid is deoxyribonucleic acid. Henceforth, we will refer to it as the "recombinant nucleic acid of the invention".

The present invention also relates to recombinant nucleic acid comprising a sequence with a percentage of identity of at least 70% with SEQ ID NO:1 and a sequence with a percentage of identity of at least 70% with SEQ ID NO:2. Preferably the identity is 80%, 85%, 90%, 95%, 96%, 97%, 98%, 99% or 100%.

The present invention also relates to nucleic acid analogous to the recombinant nucleic acid of the invention. The term "analogous" refers to a region of equal function but different sequence. Therefore, it would refer to nucleic acids coding for the same recombinant protein as that encoded by the recombinant acid of the invention but in a different sequence.

In the present invention the terms "sequence" or "nucleotide sequence" are used interchangeably in the description.

The viral haemorrhagic septicaemia virus (VHSV) is a virus of the *Rhabdoviridae* family, *Novirhabdovirus* genus. VHSV_{07.71} is the strain preferably employed in the present invention.

A preferred embodiment of the first aspect of the invention relates to recombinant nucleic acid wherein the subunit (a) consists of SEQ ID NO: 1 and the subunit (b) consists of SEQ ID NO: 2.

The glycoprotein G (gpG) of viral haemorrhagic septicaemia virus (VHSV) is a protein anchored to the membrane of the virus, with GenBank accession number A10182. The ectodomain of the glycoprotein G of the viral haemorrhagic septicaemia virus (VHSV) is encoded by sequence SEQ ID NO: 1, and corresponds to amino acids 20-462 inclusive, of the VHSV glycoprotein G (amino acid sequence collected in SEQ ID NO: 5). GpG is preferably from the VHSV_{07.71} strain but gpG from other VHSV strains can be used such as VHSV860 (GenBank accession number AY546628.1 of) with an identity between gpGs of 91%).

Pleurocidin is an antimicrobial cationic peptide belonging to the family of antimicrobial peptides present in Flounder. It was first isolated in the mucus of Winter Flounder (*Pseudopleronectes americanus*). It has also been found in other flatfish such as American plaice (*Hippoglossoides platessoide*), Atlantic halibut (*Hipoglossus hipoglossus*) or yellowtail flounder (*Limanda ferruginea*). Pleurocidin has a broad spectrum activity against bacteria and also participates in the innate defence of the host. The mature peptide is made up of 25 amino acids, and the prepropeptide of 60. The first 22 amino acids of the prepeptide correspond to the pleurocidin signal peptide, sequence included in the construct. SEQ ID NO: 2 corresponds to nucleotides 141-209 inclusive of the coding sequence for pleurocin in flounders (*Limanda limanda*) (GenBank accession number DQ248966) with amino acid sequence contained in SEQ ID NO: 6. The pleurocidin is preferably from *Limanda limanda* but can also be the pleurocidin of other fish, such as *Pseudopleronectes americanus, Hippoglossoides platessoide, hipoglossus hipoglossus or Limanda ferruginea.*

An even more preferred embodiment of the first aspect of the invention relates to recombinant nucleic acid which further comprises the promoter of β-actin of carp (pAE6) of sequence SEQ ID NO: 3 linked to the 5' end of the subunit (b). The regulatory sequence (promoter) of 2533 base pairs of pAE6 includes the promoter / enhancer sequences and the first exon (non-encoding) and the first intron of the β-actin sequence of carp (GenBank accession number M24113).

Another even more preferred embodiment of the first aspect of the invention relates to recombinant nucleic acid of sequence SEQ ID NO:4. This sequence corresponds to SEQ ID NO: 3 linked at its 3' end to SEQ ID NO: 2 which in turn is linked at its 3' end to SEQ ID NO: 1.

A second aspect of the invention relates to an expression cassette comprising the recombinant nucleic acid of the first aspect of the invention operably linked to transcription control elements.

The expression cassette comprises the elements necessary for expression of the nucleic acid of the invention, being operably linked to transcription and / or translation control elements.

A third aspect of the invention relates to a recombinant vector comprising the recombinant nucleic acid of the first aspect of the invention or the expression cassette of the second aspect of the invention. Said vector can comprise a promoter that directs the transcription of the nucleic acid sequence of the invention, together with other sequences to regulate transcription such as, for example, initiation and termination signals, cleavage sites, or polyadenylation signal.

A preferred embodiment of the third aspect of the invention relates to the vector wherein said vector is a plasmid.

A fourth aspect of the invention relates to a host cell comprising the recombinant nucleic acid of the first aspect of the invention, or the expression cassette of the second aspect of the invention or the recombinant vector of the third aspect of the invention. The introduction of such gene constructs can be performed with methods known in the prior art.

A preferred embodiment of the fourth aspect of the invention relates to a host cell wherein said cell is the cell of a mammal, of vertebrates, insects, yeast or bacteria. A further preferred embodiment relates to the cell wherein said cell is the cell of a mammal, of insects, yeast or bacteria.

A fifth aspect of the invention relates to a fusion protein generated by translation of the recombinant nucleic acid of the first aspect of the invention.

A sixth aspect of the invention relates to a method of producing the fusion protein of the fifth aspect of the invention comprising the culturing of the host cell of the fourth aspect of the invention under suitable conditions for expression of the sequence encoded by the recombinant nucleic acid of the first aspect of the invention.

A seventh aspect of the invention relates to the use of the recombinant nucleic acid of the first aspect of the invention, or of the expression cassette of the second aspect of the invention or of the vector of the third aspect of the invention, for the preparation of a pharmaceutical composition for treatment and / or prevention of haemorrhagic septicaemia caused by viral haemorrhagic septicaemia virus (VHSV) in fish. Therefore, it also refers to the use of the recombinant nucleic acid of the first aspect of the invention, or of the expression cassette of the second aspect of the invention, or of the vector of the third aspect of the invention, for the treatment and / or prevention of haemorrhagic septicaemia caused by VHSV.

In the present invention the term "fish" refers to vertebrate aquatic organisms likely to suffer from haemorrhagic septicaemia caused by VHSV, preferably fish belonging to the *Salmonidae* family, such as trout or salmon.

The term "pharmaceutical composition" herein refers to any substance used for the prevention, diagnosis, alleviation, treatment or cure of viral haemorrhagic septicaemia caused by VHSV. The pharmaceutical composition of the invention can be used either alone or in combination with other pharmaceutical compositions, including vaccines and antiviral drugs. The combination of said pharmaceutical composition with vaccines or antiviral drugs could make the immune response generated more effective, thus acting as an adjuvant. The term pharmaceutical composition and medicament are used in this invention interchangeably.

In another preferred embodiment, the pharmaceutical composition referred to herein is a vaccine. In the context of the present invention the term "vaccine" refers to a preparation used to elicit an immune response to the "viral haemorrhagic septicaemia" disease caused by VHSV. The vaccine of the invention comprises the recombinant DNA of the invention. It is a preparation which, once inside the organism, translates into a recombinant protein and causes the response of the immune system by antibody production, and generates immunological memory producing permanent or temporary immunity.

In the present invention the term "antiviral" refers to any substance that does not permit virus replication, assembly or release.

An eighth aspect of the invention relates to a vaccine comprising a therapeutically effective amount of the recombinant nucleic acid of the first aspect of the invention or of the expression cassette of the second aspect of the invention or of the vector of the third aspect of the invention.

A preferred embodiment of the eighth aspect of the invention relates to the vaccine further comprising another active ingredient, such as other components of multivalent vaccines that confer protection against other pathogens, such as bacterins, bacterial lipopolysaccharide, beta-glucans, 1-3, chitosan, levamisole, and other inflammatory agents.

In the present invention the vaccine comprises a therapeutically effective amount of the nucleic acid of the invention.

As used herein, the term "therapeutically effective amount" refers to the number of modulating agents calculated to produce the desired effect and will generally be determined, among other causes, by the characteristics of such agents (and constructs) and the therapeutic effect to be achieved. The pharmaceutically acceptable adjuvants and carriers that may be used in these compositions are those known to those skilled in the art.

As used herein, the term "active ingredient" ("active substance", "pharmaceutically active substance", "active ingredient" or "pharmaceutically active ingredient") means any component that potentially provides pharmacological activity or other effect in the diagnosis, cure, mitigation, treatment, or prevention of disease, or affect the structure or function of the body of man or other animals. The term includes those components that promote a chemical change in the development of the drug and are present in the same in an established modified form that provides the specific activity or effect.

Another preferred embodiment of the eighth aspect of the invention relates to the vaccine which further comprises at least one adjuvant, preferably a non-mineral oil adjuvant, such as Montanide ™ (Montanide ™ ISA for example).

Another even more preferred embodiment of the eighth aspect of the invention relates to the vaccine further comprising at least one excipient and / or at least one carrier, both being pharmaceutically acceptable

The term "excipient" refers to a substance that helps the absorption of the pharmaceutical composition, preferably a vaccine, comprising the nucleic acid of the invention, stabilizing it or helping in its preparation in the sense of giving it consistency, shape, flavour or any other specific functional feature. Thus, the excipients may have the function of holding the ingredients together such as starch, sugar or cellulose, a sweetening function, dye function, protection function of the medicament to insulate it from the air and / or humidity for instance, as filler of a tablet, capsule or any other form of presentation such as dibasic calcium phosphate, a disintegrating function to facilitate dissolution of the components and their absorption in the intestine, without excluding other excipients not listed in this paragraph.

"Pharmacologically acceptable" (or "pharmaceutically acceptable") carrier refers to those substances or combination of substances known in the pharmaceutical industry, used in the manufacture of pharmaceutical dosage forms and includes, but is not limited to, solids, liquids, solvents or surfactants. The carrier can be an inert substance or having a similar action to any of the compounds of the present invention and whose function is to facilitate the incorporation of the drug as well as of other compounds, to allow better dosage and administration or to give consistency and shape to the pharmaceutical composition. When the presentation is liquid, the carrier is the diluent. The term "pharmacologically acceptable" means that the compound referenced is permitted and has been evaluated such that no harm is caused to the organisms to which it is administered.

The pharmaceutical composition or medicament provided by this invention can be provided by any route of administration, for which said composition is formulated in the pharmaceutical form suitable to the chosen route of administration.

In the present invention the usefulness of the vaccine of the invention has been demonstrated for both intraperitoneal and intramuscular administration, for this reason, another even more preferred embodiment of the eighth aspect of the invention relates to the vaccine formulated for intraperitoneal or intramuscular administration.

A ninth aspect of the invention relates to the recombinant nucleic acid of the first aspect of the invention or to the expression cassette of the second aspect of the invention or to the recombinant vector of the third aspect of the invention or to the recombinant protein of the fifth aspect of the invention or to the vaccine of the eighth aspect of the invention for use in the treatment and / or prevention of haemorrhagic septicaemia caused by VHSV in fish. In a preferred embodiment the fish belong to the *Salmonidae* family, such as trout or salmon.

Throughout the description and claims the word "comprises" and its variants are not intended to exclude other technical features, additives, components or steps. For those skilled in the art, other objects, advantages and features of the invention will become apparent partly from the description and partly from the practice of the invention. The following examples and figures are provided by way of illustration, and are not intended to be limiting of the scope of the present invention.

### BRIEF DESCRIPTION OF THE DRAWINGS

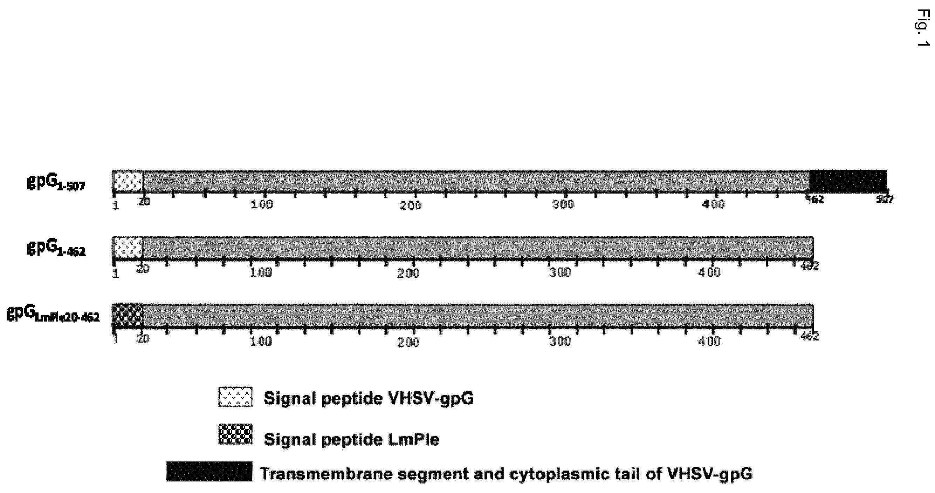

**Figure 1****. Diagram of proteins encoding plasmid constructs used in the invention.** The constructs of VHSV-gpG are shown with the amino terminus on the left. gpG1-507, entire sequence of VHSV-gpG. Residues 1 to 20, signal peptide of VHSV-gpG, 20 to 462, VHSV-gpG ectodomain and 462 to 507 the transmembrane domain and cytoplasmic tail of VHSV-gpG. gpG1-462 comprises the ectodomain of VHSV-gpG including the signal peptide. gpGLmPle20-462 contains the ectodomain VHSV- gpG and the signal peptide of the *Limanda limanda* Pleurocidin (LmPle).

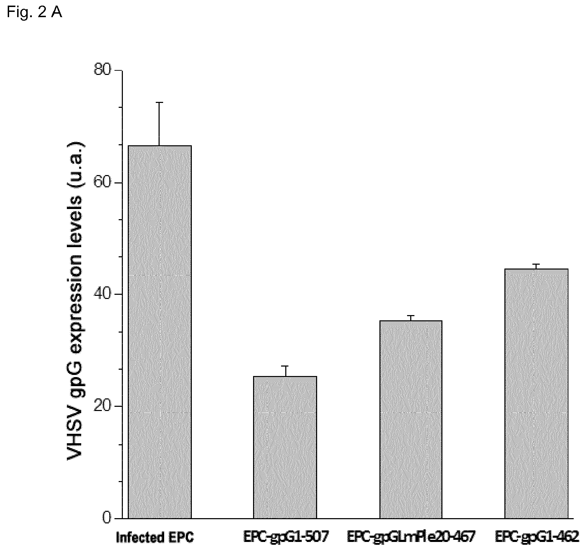

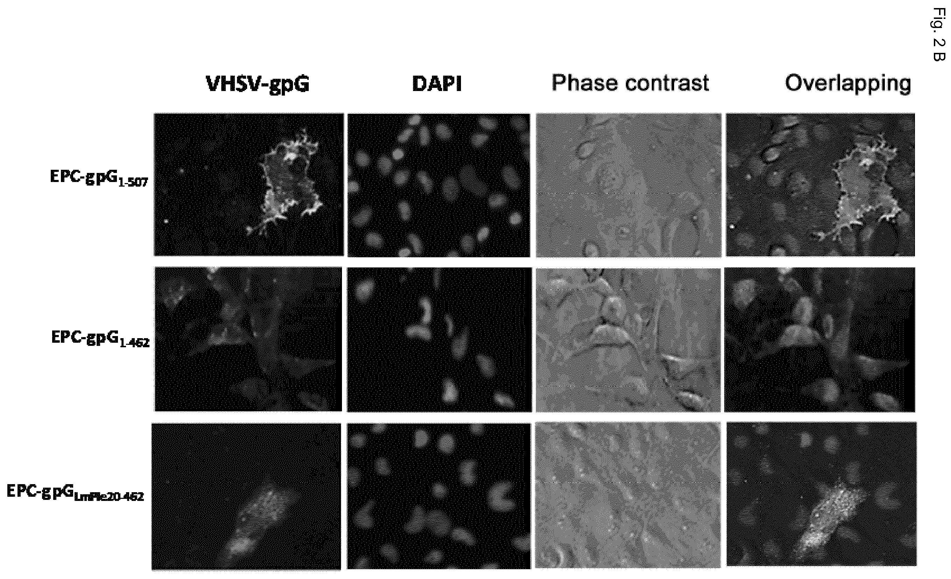

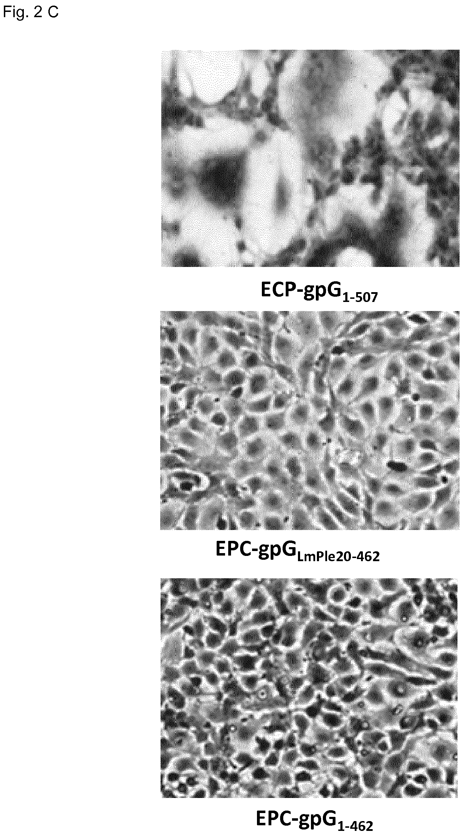

**Figure 2****. Characterization of the different forms of the VHSV-gpG protein expressed by stably transfected EPC cells.** EPC cells were cotransfected with pAE6- G1-507, pAE6-G1-462 and pAE6-GLmPle20-462 together with pAE6-pac (with the puromycin resistance gene). After the incubation period with puromycin, the presence of each of the different constructs was measured in the remaining cells by RT-PCRq (A). A shows the transcript levels of each stably transfected line with each of the constructs compared with infected EPC cells. B shows immunofluorescence using a cocktail of anti-G monoclonal antibodies and an anti-mouse IgG antibody bound to fluorescein isothiocyanate (FITC). The EPC-gpG1-507 monolayers were fixed with Citofix and EPCgpG1- 462 and EPC-gpG1-462 were permeabilized with 0.02% PBS-Triton® X. Planes are shown in the fluorescent FITC field (field 1) fluorescent DAPI (field 2), visible (field 3) and overlapping (field 4). C shows the fusion assay to evaluate the functionality of the protein at acidic pH. Cells were fixed with cold methanol, dried and stained with Giemsa (5 mg / ml in PBS).

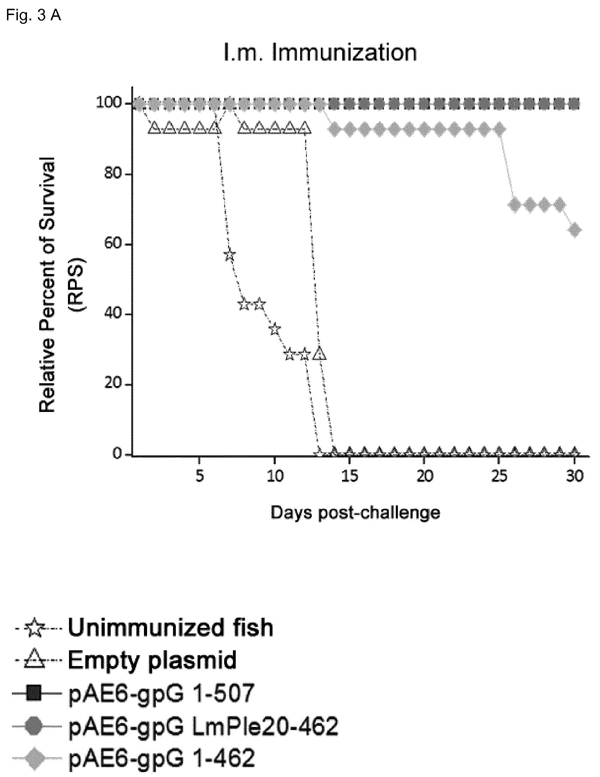

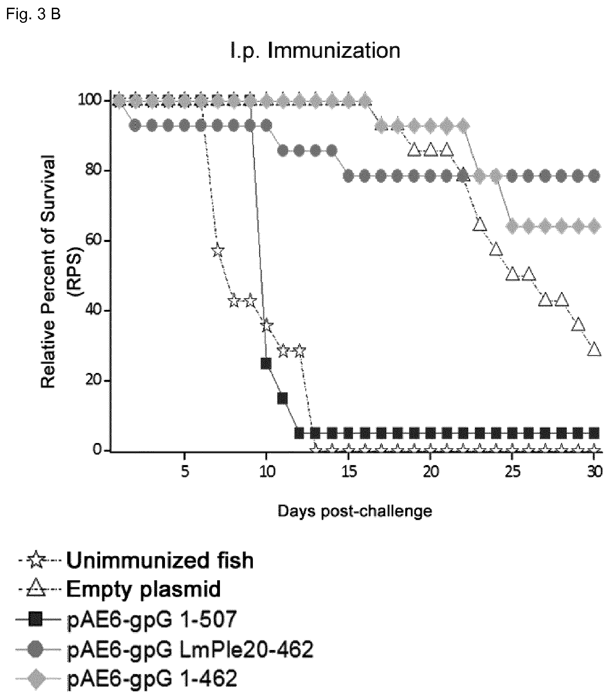

**Figure 3****. Relative Percent of survival against a challenge with VHSV.** Fish were immunized with each of the constructs intramuscularly (A) and intraperitoneally (B) in separate tanks. The challenge was carried out 40 days after immunization. Unvaccinated fish (stars), empty plasmid (triangle), pAE6- G1-507 (black square), pAE6-GLmPle20-462 (circle) and pAE6-G1-462 (diamond).

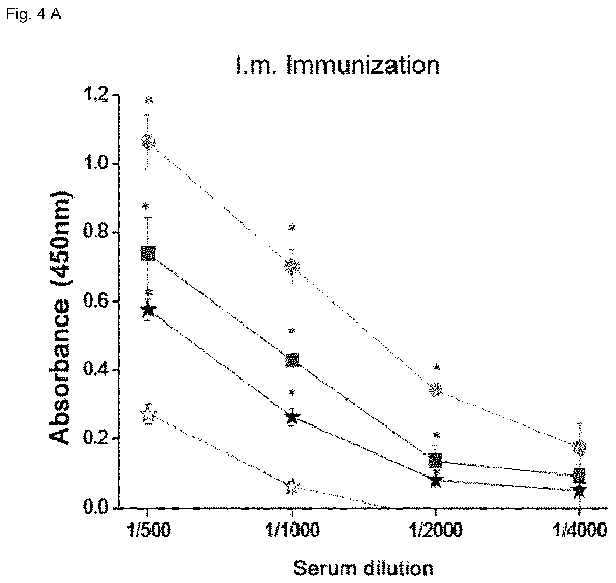

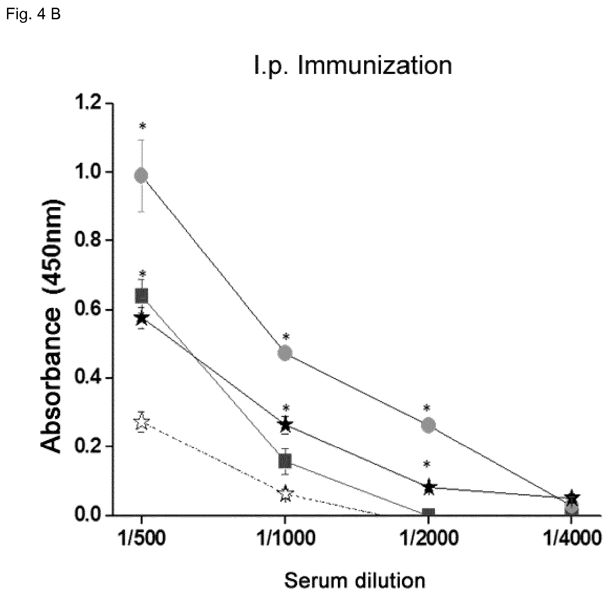

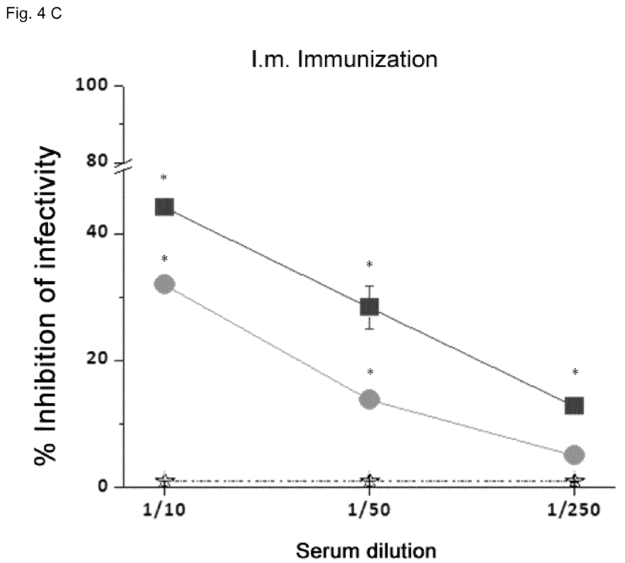

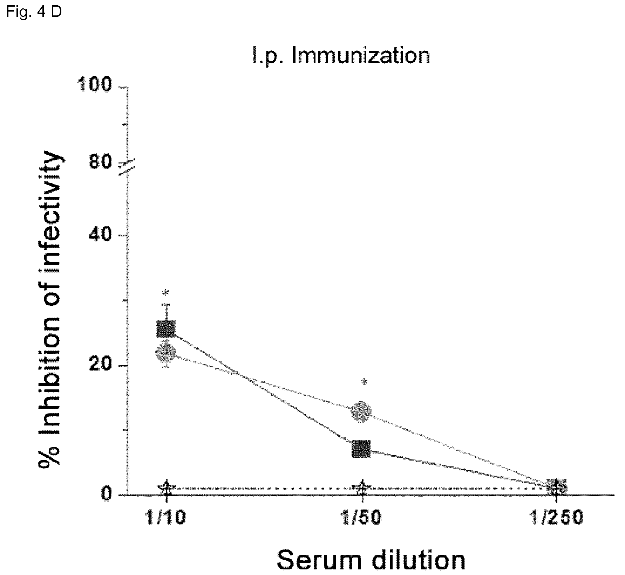

**Figure 4****. Specific IgMs anti gpG-VHSV and neutralizing antibodies in serum of immunized trout.** A, fish immunized i.m. and B, fish immunized i.p. The presence of anti VHSV-gpG antibodies in trout serum was determined 30 days post-immunization by an enzyme-linked immunosorbent assay (ELISA) using a recombinant VHSV-gpG fragment (amino acids 56-110) called fragment 11 as antigen. The serum obtained from VHSV survivor trout was used as a positive control. Absorbance was measured at 450 nm. The points represent the mean values and standard deviations of 4 fish per group, testing done in triplicate. Unimmunized fish (blank star), empty plasmid (triangle), pAE6-G1-507 (black square), pAE6-LmPle20-462 (circle), surviving fish (black star). C and D, neutralization assay of fish immunized i.m. or i.p., respectively; VHSV_{07.71} was incubated with serial serum concentrations of immunized trout or control trout. Results are expressed as percentage of inhibition relative to infected cells not treated by the formula (100 - (number of positive DAB plates in EPC treated with trout serum supplemented and infected with VHSV07.71 / number of positive DAB plates in EPC treated with serum and infected with VHSV07.71 without supplement) x 100) .The points represent the mean values and standard deviations of 4 fish per group, testing done in triplicate. Unimmunized fish (blank star), empty plasmid (triangle), pAE6- G1-507 (black square), pAE6-LmPle20-462 (circle), surviving fish (black star). Points marked with "*" showed statistically significant differences compared to control fish p <0.05.

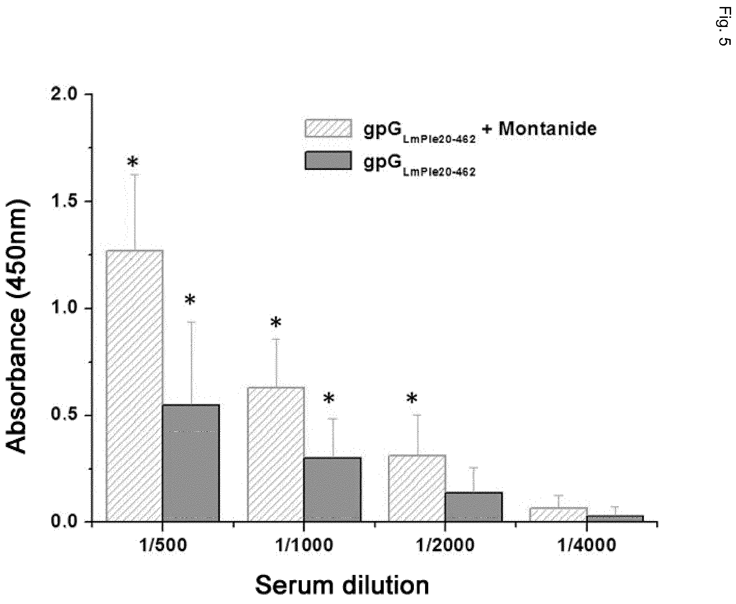

**Figure 5****. Specific IgMs anti gpG-VHSV in trout vaccinated with pAE6-GLmPle20-462 and Montanide™.** The presence of specific IgMs in trout serum against the fragment 11 in vaccinated fish at 30 days with pAE6-GLmPle20-462 or pAE6-GLmPle20-462 and Montanide ™ by an ELISA, as described in material and methods. Absorbance was measured at 450 nm. The points represent the mean values and standard deviations of 4 fish per group, testing done in triplicate. pAE6-GLmPle20-462 (dark bars), pAE6-GLmPle20-462 and Montanide ™ (hatched bars). Items marked with "*" showed statistically significant differences compared to control fish p <0.05.

### EXAMPLES

The following specific examples provided in this patent document serve to illustrate the nature of the present invention. These examples are included for illustrative purposes only and are not to be construed as limitations to the invention claimed herein. Therefore, the examples described below illustrate the invention without limiting the scope thereof.

### Example 1: Generation of the recombinant vaccine against VHSV.

The present invention relates to a recombinant DNA vaccine against VHSV comprising a recombinant deoxyribonucleic acid which comprises the coding sequence for the ectodomain of the glycoprotein G of the viral haemorrhagic septicaemia virus (VHSV), the coding sequence for a signal peptide of the pleurocidin of *Limanda limanda* and the promoter of β-actin of carp. For this purpose, the C-terminal transmembrane segment and cytoplasmic domain of the gpG of VHSV were removed, from amino acid 462 to 507, obtaining a soluble form of the gpG of VHSV. In addition, a secreted form of the protein was obtained by replacing the N-terminal signal peptide (from amino acid 1 to 20) with the pleurocidin of flounder, an antimicrobial peptide that is naturally secreted by the cell.

The results of genetic immunization showed that the DNA vaccine encoding the secretable form of gpG of VHSV was able to protect fish against infection with a lethal dose of VHSV administered both i.p. and i.m., this being the first description of a DNA vaccine against fish virus that has been successful in conferring protection using the intraperitoneal route. In addition, this optimized DNA vaccine maintains its protective ability when administered with an oily adjuvant, so it thus may be included in the formulations of multivalent vaccines used in salmonid fish farms. Moreover, the results indicate that the subcellular localization of the antigen in cells transfected *in vivo* has a major role in regulating the immunogenicity of DNA vaccines, since differences have been found in the induction of the immune response given by the secretable antigen and by the membrane-anchored antigen at short post-immunization times.

### MATERIALS AND METHODS

### Design of plasmid constructs:

Plasmid pAE6-G1-507 encoding the wild gpG of VHSV under the control of regulatory sequences 5' of the β-actin gene of carp.

Taking the complete sequence of gpG-VHSV, two new constructs were designed; i) a soluble form of the gpG, G1-462, in which the transmembrane segment and the cytoplasmic tail have been removed (Figure 1) and ii) a secreted form, gpGLmPle20-462, similar to the soluble form, but carrying the signal peptide of the antimicrobial peptide of the pleurocidin of *Limanda limanda* (DQ248966). Both constructs were synthesized by Biost (Montreal, Canada) and were cloned in plasmid pAE6 to obtain plasmids pAE6-G1-462 and pAE6-GLmPle20-462 through standard procedures.

### Cell cultures and viruses:

The EPC cell line (*papulosum cyprini* from *Pimephales promelas*), purchased from the *American Type Culture Collection* (ATCC number CRL-2872) was used in this work.

EPCs were maintained at 28 °C in an atmosphere of 5% CO₂ with RPMI-1640 medium (Gibco, *Invitrogen Corporation,* UK) containing 10% foetal calf serum (FCS) Sigma, Che. Co., St. Louis, MS, USA), 1 mM pyruvate (Gibco, *Invitrogen Corporation,* UK), 2mM Glutamine (Gibco), 50 µg / ml gentamicin (Gibco) and 2 µg / ml Fungizone.

The viral haemorrhagic septicaemia virus, strain 07.71 (VHSV 07.71) isolated in France from rainbow trout (*Oncorhynchus mykiss*), occurred in the EPC cells at 14 °C as already described above. VHSV supernatants were used to infect monolayers of EPC, and then the supernatants were clarified by centrifugation at 4000 x g for 30 min and stored aliquoted at - 80 ° C. The virus was titrated in 96 well plates using the previously developed Focus Forming Assay (focus forming units, f.f.u.).

### Generation of stably transfected EPC lines expressing the different constructs of the gpG of VHSV:

EPC cells enriched in cells stably expressing the various forms of the gpG were obtained by co-transfecting with 1.5 µg of pAE6-G1-507, pAE6-G1-462 or pAE6-GLmPle20-462 plus 0.25 µg pAE6-*pac* (puromycin resistance gene) and incubating at 20 ° C for 48h. Puromycin resistant cells were selected by adding 20 µg / ml of puromycin (Sigma) for 3 days to the culture medium and those resistant were plated into 96 well plates at different densities, adding conditioned medium of untransfected EPC cells. Then, it was evaluated whether the cells expressed the different forms of gpG by quantitative PCR using specific primers (SEQ ID NO: 7 and SEQ ID NO: 8), located in the central region of the gpG and thus recognizing the 3 forms (secretable soluble and wild). After 2 weeks, puromycin-resistant cells were transferred to 48 well plates and grown with puromycin and conditioned medium. Finally, lines EPCgpG1-502, EPC-gpG1-462 and EPC-gpG LmPle20-462 were seeded in 6 well plates and then were transferred to culture flasks. The lines were maintained for 1 year at 20 ° C in the presence of 10 µg / ml puromycin.

The specific primers used in the quantitative PCR to check whether cells expressed the different forms of the gpG are described in SEQ ID NO: 7 (forward primer or sense primer, CGACCAGCTCAACTCAGGTGTCCTCAT) and SEQ ID NO: 8 (reverse primer or antisense primer, GGTGACTCGATAAGTCACTCTGTGCAC).

### Analysis of the expression of the different forms of VHSV gpG in stably transfected EPC lines:

The expression of the various forms of gpG in the EPC cells transfected was analysed at the transcriptional level by quantitative RT-PCR. For RT-PCRq, ribonucleic acid (RNA) was extracted and cDNA was synthesized as indicated below. The quantitative PCR assays were conducted using primers specifically designed to detect the three forms of the gpG (secretable, soluble and wild) (SEQ ID NO: 7 and SEQ ID NO: 8), the internal reference to normalize data being the 18S rRNA gene (18S ribosomal RNA, *Human 18S RNA* ref. 4310893E, *Life Technologies*).

### Detection of the localization of the various forms of the VHSV gpG by immunofluorescence (IF) in stably transfected EPC cultures:

In order to detect the presence of the various forms of gpG, the cells were grown in 96 well plates and fixed with 4% paraformaldehyde (15 min at room temperature) and left untreated or 0.02% Triton ® X100 in PBS was added to permeabilize. Then the cells were incubated with a cocktail of anti gpG monoclonal antibodies, diluted 200 times for 2 h at room temperature. After washing with PBS, 100 µl of a mouse IgG fluorescently labelled antibody (Sigma) diluted 200 times was added to each well for 45 min. To visualize nuclei, it was incubated with 0.1 mg / ml DAPI (Sigma) for 10 min. The stained cells were visualized and photographed with an inverted fluorescence microscope (Nikon Eclipse TE2000-U, Nikon Instruments, Inc., NY) equipped with a digital camera (Nikon DS-1QM).

### Fusion assays

Both the EPC and stable lines were grown in 96 well plates and after 48 hours the medium was replaced with fusion medium at pH 6 for 30 min at 14 ° C. Monolayers were then incubated with fusion medium at pH 7.5 for 2 h at room temperature. To assess the fusion, the cells were fixed with cold methanol, allowed to dry and stained with Giemsa (5 mg / ml in PBS). The cells were photographed with an inverted fluorescence microscope (Nikon Eclipse TE2000-U, Nikon Instruments, Inc., NY) equipped with a digital camera (Nikon DS-1QM). As a positive control of fusion, VHSV-infected EPC cells were used.

### Fish:

Rainbow trout (*Oncorhynchus mykiss*) measuring 5-6 cm obtained from a VHSV-free fish farm (Lillogen, Leon, Spain) were maintained in 50-L tanks on the premises of the Universidad Miguel Hernández of Elche (UMH) at 12 to 14 °C in a system of recirculating dechlorinated water and fed daily using a commercial diet (Trucha, Leon, Spain). Prior to the experiments, fish were acclimated to the laboratory conditions for 2 weeks. The animals were treated according to national and European guidelines and regulations on animal care. Work with animals was approved by the ethics committee of the UMH (authorization IBM-AE-001-11).

### DNA immunization protocol:

Immunization experiments with recombinant DNA were carried out as follows; fish were anesthetized by immersion in a buffer containing 50 µg / ml tricaine (MS-222; Sigma) before injection and division into the corresponding groups. Two independent immunization tests were conducted, the first: to test the protection afforded by the various DNA vaccines, groups of 30 fish (Table 1) were intraperitoneally (ip) or intramuscularly (im) immunized with 50 µl of PBS (unimmunized control trout) or 50 µl of PBS with 2 µg of pAE6, pAE6-G1-502, pAE6-G1-462 or pAE6-GLmPle20-462. Then cells from the peritoneal cavity were collected three days post-immunization. To this end, the mucus was wiped from the abdominal part of the fish with ethanol, then, to prevent the cells from the peritoneal cavity being contaminated by blood, the fish were bled via the dorsal aorta. Then, using a 25 gauge needle, 300 µl of PBS was injected i.p. in the ventral line and was massaged for 30 seconds to disperse the cells of the peritoneum in the injected PBS. Then, the PBS with the cells were collected with the same syringe and put into the RNA lysis buffer until processing.

**Table 1. Immunization assay in groups of 30 fish immunized intraperitoneally (i.p.) or intramuscularly (i.m.).**

| | **FIRST ASSAY** | | **SECOND ASSAY** | |
|---|---|---|---|---|
| Route of administration | i.m. | i.p. | i.m. | i.p. |
| | PBS | PBS | | PBS |
| | Empty plasmid | Empty plasmid | pAE6-G₁₋₅₀₇ | Empty plasmid |
| Plasmid | pAE6-G₁₋₅₀₇ | pAE6-G₁₋₅₀₇ | | Empty plasmid + adjuvant |
| | pAE6-G₁₋₄₆₂ | pAE6-G₁₋₄₆₂ | | PBS + adjuvant |
| | pAE6- | pAE6- | | pAE6-GLmPle20-462 |
| | G_{LmPle20-462} | G_{LmPle20-462} | | |
| | | | | pAE6-G_{LmPle20-462}⁺ adjuvant |

For the second assay, it was tested whether the DNA vaccine encoding the secreted form of gpG could be administered together with conventional adjuvants. For this purpose, groups of 30 fish (Table 1) were injected i.p. with 50 µl PBS, 50 µl PBS with 2 µg of pAE6 or 2 µg of pAE6-GLmPle20-462 in the presence or absence of non-mineral oil adjuvant Montanide™ ISA 763 AVG (Seppic, France) in a 30:70 ratio respectively. In order to obtain a fluid emulsion in the presence of Montanide ™, manufacturer's recommendations were followed. As control of the experiment, fish used were i.m. immunized with the wild form of the protein. In both assays, at day 30 post-immunization blood was extracted from the caudal vein (4 fish per group) to analyse the presence of antibodies and their neutralizing activity.

### Isolation of RNA and cDNA synthesis:

The *E.Z.N.A.*® *Total RNA* kit (Omega Bio-Tek, Inc., USA) was used for RNA extraction following the manufacturer's instructions. Isolated RNA was stored at - 80 °C until use. One microgram of RNA estimated by the spectrophotometer NanoDrop™ 200 c (Thermo FisherScientific Inc.), was used to obtain cDNA using reverse transcriptase M-MLV (Invitrogen) as traditional procedures.

### Quantitative PCR assays:

PCRq assays (chain reaction of quantitative polymerase) were conducted using the system ABI PRISM® 7300 (Applied Biosystems, NJ, USA) and SYBR® green. To detect the various forms of the VHSV gpG both in the stably transfected EPC cells and in cells of the peritoneal cavity, specific primers (SEQ ID NO: 7 and SEQ ID NO: 8) were designed. Expression was analysed by the method of 2-ΔCt wherein ΔCt is determined by subtracting the value of Ct of 18S rRNA, used as endogenous control, from the value of the target Ct. For *in vivo* expression, primers are those described in SEQ ID NO: 7 and SEQ ID NO: 8. The cDNA of the intraperitoneal cavity of cells injected with empty plasmid served as a calibrator and fold increase was calculated relative to the levels of these fish. Reactions were carried out in a volume of 20 µl, wherein there was 2 µl cDNA, 900 nM of each primer, 200 nM probe, and 10 µl of the universal master mix TaqMan® (Applied Biosystems) or SYBR® green. The cycle conditions were 50 °C for 2 min and 95 °C for 10 min followed by 40 cycles at 95 °C for 15 s and at 60 °C for 1 min.

### VHSV challenge:

Forty (first immunization experiment) or 60 (second immunization experiment) days after DNA immunization, all trout remaining in each tank (15 to 20 per group) were challenged by immersion in a bath with infective VHSV (3x10⁶ TCID50 VHSV - 07.71 / ml). Mortality was recorded daily for 30 days and the relative percent of survival (RPS) was calculated by the formula: RPS = [1 - (% mortality immunized trout / % mortality control trout)] X 100.

### Estimation of specific anti-gpG-VHSV antibodies:

The presence of specific antibodies against gpG-VHSV was determined 30 days post-immunization by means of an enzyme-linked immunosorbent assay (ELISA) using the recombinant fragment of gpG (amino acids 56-110) called fragment 11 as antigen in solid phase. Serum pools (of three trouts) injected with PBS, or with different plasmid constructs, were analysed by ELISA using a monoclonal anti IgM antibody, the 1 G7. As a positive control, serum of trout surviving VHSV infection was used.

### Neutralizing activity of serum:

The presence of neutralizing antibodies was determined at day 30 post-immunization. Briefly, 10 µl of serial dilutions of trout serum in culture medium without serum Opti-Mem ™ (Opti-Mem, Invitrogen), starting with the dilution 1:10, were incubated at 37 ° C to deactivate the complement and mixed with 1.5 µl of trout complement (serum of healthy trout) in 96 well plates (30 min at 14 °C). Fifty µl of a dilution of VHSV adjusted to 1x10⁴ f.f.u./ml were added to the EPC monolayers and plates were incubated for 2 h at 14 °C. After incubation, each mixture of virus with supplemented serum was added to the EPC monolayers in 96 well plates to a final volume of 60 µl. At two hours post infection at 14 °C, infected cells were washed and 100 µl of fresh medium (RPMI Dutch modified) supplemented with 2% FCS were added and incubated for 24 h at 14 °C. To assess the infectivity of VHSV, EPC monolayers were fixed with cold methanol for 10 min and allowed to dry. The monoclonal antibody 2C9 directed to the N protein of VHSV diluted 1000 times in dilution buffer (0.24mM merthiolate, 5 g / I Tween 20 ™, 50 mg / L phenol red in PBS, pH 6.8) was added to the wells ( 100 µl / well) and incubated for 1 h at room temperature. An antibody conjugated with anti IgG mouse peroxidase (100 µl) (Nordic, Tilburg, Netherlands) was added at a 1/300 dilution to each well for 30 min. After washing by immersion in distilled water, 50 µl at1 mg / ml diaminobenzidine (DAB) (Sigma) in PBS with H₂O₂ were added to each well. The reaction produced brown foci detectable with an inverted microscope (Nikon Eclipse TE2000-U, Nikon Instruments Inc., NY, USA). Foci stained with DAB (VHSV infected cells) were counted with a 10x grid. The results were expressed as inhibition of the infectivity of VHSV with the following formula, 100 - (number of positive foci of DAB in cultures inoculated with or without serum, supplemented and VHSV / number of positive foci of DAB in cultures inoculated with trout serum and VHSV in the absence of supplement x100).

### Statistical analysis:

To determine the statistical differences between groups and to compare between them, a Student t test or ANOVA followed by Tukey's test was used.

### Example 1.1: Characterization of the various forms of VHSV gpG in stably transfected EPC cells.

To avoid variability related to levels of transgene expression subsequent to the transfection assays, the EPC cells were enriched in the stable expression of the different constructs of the gpG (EPC-gpG1-507, EPC-gpG1-462 and EPC-gpGLmPle20-462). Expression levels of each of the constructs was measured at transcript and protein level and compared with those obtained from VHSV-infected EPC cells.

Transcripts of the three constructs were detected by RT-PCRq indicating the correct transcription of the transgenes (Figure 2A). Although no significant differences between the three forms were observed, the most abundant were detected in the soluble form (gpG1-462, ≥40-fold higher than the endogenous control). Interestingly, the lower expression was obtained in the wild form (gpG1-507) (Figure 2A). Finally, the highest levels of transcripts were detected in cells infected with VHSV (about 60 times more than the endogenous control).

### Example 1.2: Expression patterns of the different forms of the VHSV gpG in stably transfected EPC cells.

Once verified that the constructs expressed correctly, the expression pattern thereof was then studied. An immunofluorescence test was carried out in which wild gpG (gpG1-507, EPC-gpG1-507) was located in the cell membrane (Figure 2B, EPC-gpG1-507) and conversely neither of the other forms (EPC- gpGLmPle20-462 and EPC-gpG1-462), soluble nor secreted, were found in the membrane. However, these two forms (EPC-gpGLmPle20-462 and EPC-gpG1-462) were detected in the cytoplasm of cells, showing different expression patterns to those of the wild gpG (Figure 2B, 1 and 4 lower and centre panels). In the case of the secreted form (gpGLmPle20-462), the cells showed a non-uniform distribution in cytoplasmic granules, (Figure 2B, EPC-gpGLmPle20-462) resembling secretory vesicles. These cytoplasmic granules were not observed in cells expressing the soluble form (gpG1-462), in which fluorescence was distributed uniformly throughout the cytoplasm (Figure 2B, EPC-gpG1-462).

Finally, to corroborate the location and functionality of the constructs, a membrane fusion assay at acidic pH was carried out (Figure 2C). It was observed that syncytia only appeared in cells expressing wild gpG (Figure 2C, upper panel).

### Example 1.3: DNA immunization and challenges with VHSV.

Immunized and unimmunized fish were challenged with infection by immersion at 40 days post-immunization using 3x10⁶ TCID50 per VHSV-_{07.71} fish. Figure 3 shows the relative percent of survival (RPS) of the groups immunized intramuscularly, i.m. (A) or intraperitoneally, i.p. (B) with each of the constructs (Table 1).

Significant protection was observed with RPS values of 80 to 100% in fish immunized with plasmid encoding the secreted form of the gpG (pAE6- GLmPle20-462), regardless of the route of administration used (100% by im and 80 % by ip) (Figure 3A and B, circles, note the for im injection, the circles are above the squares at 100% protection). By contrast, construct with the wild form (pAE6- gpG1-507, dark squares) only protected 100% when the fish were immunized intramuscularly. Furthermore, levels of protection observed in i.m. and i.p. with the soluble form of the gpG (pAE6-gpG1-462, diamonds) were in both cases 60%. As expected, unimmunized control fish receiving PBS or empty plasmid (Figure 3A and B, stars and triangles, respectively), had an RPS of about 0% (Fig. 3A and B). All dead fish presented bleeding in the abdominal cavity, typical symptom of VHSV infection (data not shown).

### Example 1.4: The presence of specific antibodies with neutralizing activity in serum of trout immunized with pAE6-GLmPle20-462 and DAE6-QDG1-507.

Blood samples were collected 30 days post-immunization to analyse whether the protection levels obtained by pAE6- GLmPle20-462 and pAE6- gpG1-507 correlated with the levels of specific antibodies against gpG. As a control, a pool of serum from trout resistant to VHSV infection was used.

Samples of fish vaccinated with each of the constructs by both routes of administration were tested for the presence of specific antibodies (Figure 4A and B). The antibody titre of immunized fish (squares and circles, Figure 4) were similar to those obtained in the serum of fish surviving the infection (black star, Figure 4A and B). The highest specific antibody titre was found in trout immunized i.m. or i.p. with the secretable form of gpG, pAE6-GLmPle20-462. Antibody levels were higher in those immunized i.m., for both constructs (Figure 4A and B). The neutralizing capacity of antibodies present in the serum of immunized fish was analysed using an ELISA developed for detecting VHSV _{07.71} strain. None of the sera tested showed effective neutralizing activity, since inhibition of VHSV infectivity after neutralization reached a maximum of 55% in the fish immunized with wild gpG at the minimum dilution used (1/10) (Figure 4C and D).

### Example 1.5: Effect of Montanide ™, a commercial adjuvant for fish vaccines in the protective response induced by i.p. immunization with plasmid pAE6-GLmPle20-462.

Since the secreted form was the only one capable of conferring protection both via the i.m. and i.p. routes, an oil adjuvant was used to combine it with the plasmid pAE6- GLmPle20-462. The pAE6- GLmPle20-462, empty plasmid or PBS were mixed with non-mineral adjuvant Montanide ™. Once injected via the i.p. route, fish were challenged with a lethal dose of VHSV 60 days post-immunization. The presence of specific antibodies in serum of immunized fish at 30 days post-immunization show a clear increase in the group injected with plasmid together with Montanide ™ (Figure 5). However, the RPS values obtained were similar (∼ 75%) (data not shown) for fish immunized with pAE6- GLmPle20-462 with or without Montanide ™. No injuries or delays in fish growth were observed during this study.

### Discussion

Despite the vast amount of knowledge about DNA vaccines accumulated in the last 15 years, the mechanism of activation / primacy of the various methods of administration has not been researched in depth. Therefore, it is not possible to choose the most appropriate method of administration in each case, which limits the versatility of DNA vaccines. In the present invention, using a DNA vaccine based on the glycoprotein gene of VHSV, the subcellular localization of the antigen encoded by the plasmid in transfected cells *in vivo* was modified to solve the problems of conferred protection related with the administration route of the vaccine. To this end, and based on a plasmid encoding the wild form of VHSV-gpG (membrane-anchored protein), plasmids were designed encoding a soluble form (pAE6-G1-462) or secreted soluble form (pAE6-GLmPle20-462) of the viral protein. Soluble forms of surface glycoproteins of other viruses, such as rabies virus or human cytomegalovirus, have been used in order to increase the immunogenicity of vaccines. However, replacing the signal peptide of a viral glycoprotein with the signal peptide of an antimicrobial peptide that is secreted naturally, pleurocidin, is a novel strategy that had not been previously tested. The signal peptide of pleurocidin was chosen based on the abundance of this peptide in the mucous secretions of the skin in fish, indicating an efficient mechanism of secretion in these species.

After determining the correct expression and subcellular localization of the different forms of VHSV-gpG *in vitro,* the fish were immunized intramuscularly and intraperitoneally with one of the three forms of VHSV-gpG (membrane-anchored form, soluble or secreted soluble) and the protection rates after a countertest with VHSV were recorded. The plasmid encoding the secreted soluble form (pAE6-GLmPle20-462) was the only one conferring protection by both routes, intramuscular and intraperitoneal, this being the first description of a DNA vaccine that confers protection when administered intraperitoneally. In addition, protection rates correlated with the levels of specific antibodies against VHSV-gpG, the antibody titres being higher in all fish immunized with pAE6-GLmPle20-462, regardless of the route of administration. Moreover, differences in the antibody response generated by the different forms of VHSV-gpG were not due to different expression levels produced, as the three forms were included in the same DNA skeleton (vector) and produced similar RNA levels *in vivo.* Therefore, and as described for other proteins, the secreted form of the antigen induces the largest responses following DNA immunization.

A plausible explanation of why the secreted form of VHSV-gpG induces higher antibody response than the other forms is that the differences observed are due to the availability of the antibody for the activation of B cells. In addition to the potential role of B cells, and as previously shown, the different localization of the antigen could influence induction and preferential proliferation of different subsets of T helper cells. To check this, the presence of B and T cells in the peritoneal cavity at 3 days post-immunization was analysed by assessing transcription levels of specific markers of B and T cells. In general, the results show an increase in the infiltration of both B and T cells in response to the soluble secreted form of VHSV-gpG at a short post immunization time. With respect to the B cells, whereas cells expressing the membrane form of IgM was the predominant subpopulation of B cells in fish immunized with pAE6-gpG1-507, cells expressing membrane igt were the most abundant in fish immunized with pAE6-GLmPle20-462. Similarly, specific antibodies and high levels of igt were previously detected in the liver of fish immunized intramuscularly with pAE6-G1-507. In general terms, the results suggest a direct implication of B cells, and particularly a B cell subtype expressing the membrane form of igt, in the organization of the protective immune response to short post-immunization times. Importantly, the B cells of fish have phagocytic ability *in vivo,* suggesting an important role of these cells in the communication between innate and adaptive immune processes. Therefore, B cells in fish are likely to play a greater role in DNA vaccination compared to mammals. On the other hand, a clear polarization of the immune response to Th1 was observed *in vivo* for the secreted form of the gpG.

Since the most commonly used adjuvants in vaccines administered intraperitoneally are oily solutions, the effect of the oil adjuvant Montanide ™ ISA 763 AVG was evaluated, in response induced by i.p. injection together with the plasmid of the secretable gpG (pAE6-gpGLmPle20-462). The results show that the protection rates conferred by the DNA vaccine are similar despite the presence of Montanide ™. Since this type of adjuvant is characterized by increasing and prolonging the humoral response, antibody levels found for fish immunized with pAE6-gpGLmPle20-462 jointly with the adjuvant were higher than those obtained with the plasmid alone.

These results prove that DNA vaccines for salmonids described herein, which not only confer protection by i.m. injection, could be administered in combination with other vaccines and adjuvants, generating multivalent vaccines administered intraperitoneally. This can increase the versatility of DNA vaccines against rhabdovirus, which is of great interest to the aquaculture industry.

## Claims

1. Recombinant nucleic acid that comprises a subunit (a) comprising the sequence SEQ ID NO: 1 and a subunit (b) comprising the sequence SEQ ID NO: 2 linked to the 5' end of the subunit (a).

2. Recombinant nucleic acid according to claim 1 wherein the subunit (a) consists of SEQ ID NO: 1 and the subunit (b) consists of SEQ ID NO: 2.

3. Recombinant nucleic acid according to any one of claims 1 or 2 further comprising the promoter of β-actin of the sequence SEQ ID NO: 3 linked to the 5 'end of the subunit (b).

4. Recombinant nucleic acid according to any one of claims 1 to 3 having the sequence SEQ ID N: 4.

5. Expression cassette that comprises the recombinant nucleic acid according to any one of claims 1 to 4 operably linked to transcription control elements.

6. Recombinant vector that comprises the recombinant nucleic acid according to any one of claims 1 to 4 or the expression cassette according to claim 5.

7. The vector of claim 6 wherein said vector is a plasmid.

8. A host cell that comprises the recombinant nucleic acid according to any one of claims 1 to 4, or the expression cassette according to claim 5 or the recombinant vector according to any one of claims 6 or 7.

9. A host cell according to claim 8 wherein the cell is a mammal, insect, yeast or bacteria cell.

10. Fusion protein that is generated by translation of the recombinant nucleic acid according to any one of claims 1 to 4.

11. A method for producing the fusion protein according to claim 10 comprising culturing the host cell according to any one of claims 8 or 9 under conditions suitable for expression of the sequence encoded by the recombinant nucleic acid according to any one of claims 1 to 4.

12. Use of the recombinant nucleic acid according to any one of claims 1 to 4, or of the expression cassette according to claim 5 or of the vector according to any one of claim 6 or 7 for the preparation of a pharmaceutical composition for the treatment and / or prevention of haemorrhagic septicaemia caused by the viral haemorrhagic septicaemia virus in fish.

13. Use according to claim 12 wherein the fish belong to the *Salmonidae* family.

14. Use according to any one of claims 12 or 13 wherein the pharmaceutical composition is a vaccine.

15. A vaccine that comprises a therapeutically effective amount of the recombinant nucleic acid according to any one of claims 1 to 4 or of the expression cassette according to claim 5 or of the vector according to any one of claims 6 or 7.

16. The vaccine according to claim 15 further comprising another active ingredient.

17. The vaccine according to any one of claims 15 or 16 further comprising at least one adjuvant.

18. The vaccine according to any one of claims 15 to 17 further comprising at least one excipient and / or at least one carrier, both being pharmaceutically acceptable.

19. Vaccine according to any one of claims 15 to 18 formulated for intraperitoneal or intramuscular administration.
